# EUROPEAN PATENT SPECIFICATION

(11) **EP 2 145 645 B1**
(45) Date of publication and mention of the grant of the patent: **04.01.2012**
(21) Application number: 09165445.9
(22) Date of filing: 14.07.2009
(51) Int. Cl.: A61M 16/06, A61M 16/08

(54) **Breathing assistance apparatus**
Atemhilfsgerät
Appareil d'assistance respiratoire

(30) Priority: 18.07.2008 US 82007 P; 23.07.2008 US 82974 P; 25.07.2008 US 83554 P
(43) Date of publication of application: 20.01.2010
(73) Proprietor: Fisher & Paykel Healthcare Limited, East Tamaki Auckland 2013 (NZ)
(72) Inventor: McAuley, Alastair Edwin, Auckland (NZ); Olsen, Gregory James, Auckland (NZ); Law, Kamman, Auckland (NZ)
(74) Representative: Hoarton, Lloyd Douglas Charles

(56) References cited:
- WO-A-2005/063327
- WO-A-2006/074514
- WO-A-2007/041751
- US-A1- 2002 108 613

## Description

### BACKGROUND OF THE INVENTION

### Technical Field

The present invention relates to an apparatus for use in treating respiratory disorders such as sleep apnoea. More specifically, the present invention relates to a patient interface for use as part of an apparatus which supplies gases to a user for the treatment of respiratory disorders.

### Summary of the Prior Art

It is known to provide a flow of respiratory gases to a user via an interface such as a face mask to relieve a number of ailments - for example sleep apnea or snoring. One problem with supplying a flow of gases to a user via an interface such as a face mask is that it can be difficult to form a good seal between the mask and the face. The mask is often held in place against the user's face by headgear worn on the user's head. In use, the head gear may be over tightened so that the mask is pressed uncomfortably onto the user's face. Alternatively the headgear may be under tightened or applied to the user's head too loosely, preventing the formation of an effective seal between the mask and user's face. Also, the seal between the mask and the user's face may be broken if the mask is knocked while the user sleeps, or if the user moves while sleeping.

Prior art face masks have attempted to improve the seal between the user's face and the mask and make the sealing interface with the user more comfortable. US7,308,895 and US2007/0267017 both describe a mask assembly having a seal outer sheath and inner cushion. The inner cushion has a raised nasal bridge portion which results in a more flexible seal contact on the bridge of the user's nose. The outer sheath is attached directly to the mask body.

US2007/0267017 further describes a mask assembly having a seal outer sheath and a inner cushion, where the outer sheath has a thin section in the nasal bridge region. The thin section reduces the pressure on the nasal bridge region of the user compared to when a sheath does not have a thin section. The thin bridge region allows for different sized patients to comfortably use the mask.

WO2007/041751 describes a mask assembly with a flexible seal and rigid clip for attaching the seal to a mask body. The clip provides three retention points and the mask body includes three corresponding top hat sections. To attach the clip to the mask body a user aligns the retention points with the top hat sections to fix the clip to the mask body to retain the seal in the mask assembly.

### DISCLOSURE OF THE INVENTION

It is an object of the present invention to provide an improved patient interface, or to at least provide the industry or the public with a useful choice.

In a first aspect the present invention consists of A mask assembly (2) for use as part of an apparatus for supplying a flow of respiratory gases to a user comprising:
a mask body (30), said mask body including an inlet (22) through which said flow of respiratory gases are provided to the interior of said mask body, the inlet adapted to in use be connected to a gases conduit,
a mask seal assembly (40) comprising a flexible seal (43) and a clip (42), the clip being rigid relative to the flexible seal,
said seal having a first side and a second side, the first side of said seal being shaped to approximately match the contours of a user's face and in use substantially seal against a user's face, said second side for attachment to said clip,
said clip providing an interface extending substantially the full perimeter or periphery of the mask seal assembly for releasably attaching the mask seal assembly (40) to the mask body (30), and
one of the clip or the mask body having at least one recess (47) and the other one of the mask body or the clip having a series of bumps (48), interference between the bumps and the clip or the mask body during attachment of the clip to the mask body providing a positive snap type engagement of the clip to the mask body as the bumps locate in the at least one recess,
**characterized by**
one of the clip or the mask body having a lead-in section (49) providing deflection of the clip, or the mask body, or both, when attaching the clip to the mask body, the lead-in section extending at least 50% of the full perimeter of the clip or the mask body, and
said clip being shaped to generally follow the contours of a user's face, and a rear periphery (52) of said mask body also being correspondingly shaped to generally follow the contours of a user's face,
wherein the corresponding shape of the clip and the mask body, combined with the lead in section, provides for automatic correction of angular misalignment between the seal assembly and the mask body when fitting the seal assembly to the mask body.

Preferably the seal has a wall portion extending around the periphery of the seal assembly, the wall being approximately or substantially constant in depth.

Preferably the wall depth varies by less than around 33%.

Preferably the recesses are located immediately behind the lead-in section.

Preferably the lead-in section has a length to height ratio of 3 to 7.

Preferably the lead-in section has a length to height ratio of 5.

Preferably a cross section of the mask seal assembly at the clip has a width to height ratio of 1.5 to 2.

Preferably a cross section of the mask seal assembly at the clip has a width to height ratio of 1:8.

Preferably the clip may be squeezed at opposite perimeter portions at positions absent of a said bump to disengage the seal assembly from the mask body.

Preferably the corresponding shape of the clip and the mask body provides for automatic correction of misalignment between the seal assembly and the mask body when fitting the seal assembly to the mask body.

Preferably the corresponding shape of the clip and mask body automatically correct an angular misalignment of approximately 5 degrees.

Preferably the seal assembly attaches to the mask body in a sealed engagement, a first bearing surface of the seal bearing against a corresponding second bearing surface of the mask body.

Preferably the first bearing surface includes a raised rim, in use the raised rim being compressed against the second bearing surface.

Preferably the clip has at least one wing portion, the wing portion providing a gripping flange to assist with removing the seal assembly from the mask body.

Preferably the lead-in section extends at least 50% of the full perimeter of the seal assembly clip or the mask body.

In one embodiment, said mask body and said mask seal assembly are configured to include at least one vent path for venting the interior of the mask assembly to atmosphere, the at least one vent path configured to be at least partially concealed or at least partially visually hidden when said mask assembly is viewed from the exterior.

Preferably the seal assembly has a first bearing surface and the mask body has a corresponding second bearing surface, the first bearing surface and second bearing surface in use engaged to provide a sealed engagement between the seal assembly and the mask body except for the at least one vent path.

Preferably the at least one vent path is provided by at least one discrete portion being removed from one of the first bearing surface or the second bearing surface or both.

Preferably the mask seal assembly has a first bearing surface and the mask body has a corresponding second bearing surface, the first bearing surface and second bearing surface in use engaged to provide a sealed engagement between the seal assembly and the mask body, and
the mask body has at least one vent hole venting the interior of the mask assembly to the exterior of the mask assembly, the at least one vent hole being position adjacent to the second bearing surface of the mask body, the at least one vent hole being partially concealed by a portion of the seal assembly in use so that the at least one vent hole is partially visually hidden from the exterior of the mask assembly.

Preferably at least one vent path includes a part annular channel between the interior of the mask assembly and the at least one vent hole.

Preferably at least one vent hole is located in a bottom side portion of the mask body.

Preferably at least one vent path is a direct flow path from the interior of the mask assembly to the at least one vent hole, the direct flow path being in a substantially downward direction with the mask assembly positioned on a user's face.

In another embodiment, the mask assembly further comprises:
a connector adapted to in use connect between a separate gases conduit and the inlet, the connector having a reduced diameter portion and a full diameter portion, that end of the connector which includes the reduced diameter portion adapted to connect to the inlet and that end of the connector which includes the full diameter portion adapted to connect to the gases conduit.

Preferably the connector is an elbow having a first leg, a bend, and a second leg, the first leg being the reduced diameter portion connecting to the mask assembly, and the second leg being the full diameter portion connecting to the gases conduit.

Preferably the connector includes at least one baffle in the reduced diameter portion.

Preferably the at least one baffle extends parallel to a longitudinal axis of the reduced diameter portion.

Preferably at least one baffle extends from a first end of the reduced diameter portion to a second end of the reduced diameter portion, the first end being connected to the mask assembly.

Preferably an end portion of the at least one baffle is detached from a side wall of the reduced diameter portion of the connector at the first end.

Preferably at least one baffle is aligned approximately radially to the cross section of the reduced diameter portion.

Preferably at least one baffle has a radial height of about 3mm.

Preferably the connector includes two baffles, one said baffle being located at about position three and the other said baffle being located at about position five when considering six equally spaced positions counting from top-dead-centre, top-dead-centre being position one, with the second leg at the downwards fourth, or six o'clock position.

Preferably the reduced diameter portion has a diameter of around 20mm and the full diameter portion has a diameter of around 25mm.

Preferably the connector has a first end interfacing to the mask assembly and a second end interfacing to a conduit, the first end comprising a swivel joint or the second end comprising a swivel joint or both.

Preferably the connector has a first end interfacing to the mask assembly and a second end interfacing to a conduit, the first end comprising a ball joint or the second end comprising a ball joint or both.

In another embodiment, the mask assembly further comprises:
a connector for connecting between a separate gases conduit and the inlet, the connector being an elbow connector having a first leg, a bend, and a second leg, that end of the connector which includes the first leg connecting to the inlet and that end of the connector which includes the second leg connecting to a gases conduit, and wherein the connector includes at least one baffle in the first leg.

Preferably the first leg is a reduced diameter portion and the second leg is a full diameter portion.

Preferably the at least one baffle extends parallel to a longitudinal axis of the first leg.

Preferably the at least one baffle extends from a first end of the first leg to a second end of the first leg, the first end being connected to the mask assembly.

Preferably an end portion of the at least one baffle is detached from a side wall of the first leg of the connector at the first end.

Preferably the at least one baffle is aligned approximately radially to the cross section of the first leg.

Preferably the at least one baffle has a radial height of about 3mm.

Preferably the connector includes two baffles, one said baffle being located at about position three and the other said baffle being located at about position five when considering six equally spaced positions counting from top-dead-centre, top-dead-centre being position one, with the second leg at the downwards fourth, or six o'clock position.

Preferably the reduced diameter portion has a diameter of around 20mm and the full diameter portion has a diameter of around 25mm.

Preferably the connector has a first end interfacing to the mask assembly and a second end interfacing to a conduit, the first end comprising a swivel joint or the second end comprising a swivel joint or both.

Preferably the connector has a first end interfacing to the mask assembly and a second end interfacing to a conduit, the first end comprising a ball joint or the second end comprising a ball joint or both.

In one embodiment, the mask assembly comprises:
a rigid mask body, including an inlet through which said flow of respiratory gases are provided to an interior of said mask assembly,
a mask seal assembly coupled to the mask body in use, the mask seal assembly including a mask seal being shaped to approximately match the contours of a user's face and in use substantially seal against a user's face,
said seal comprising a thin region and a thick region, the thin region inflating under typical CPAP pressures to an elastically stretched condition, the seal material having a strain greater than 0.1 when in the elastically stretched condition.

Preferably the thin region has a thickness of 0.05mm - 0.2mm, the seal material having a Shore A hardness of around 40.

Preferably the seal material is silicone.

Preferably the seal is manufactured by injection molding in a closed mold, the point of injection into the mold being in the thin region of the seal.

Preferably the thin region is located in the nasal bridge region.

Preferably the thin region extends around a periphery of the seal, in use the periphery being in contact with a user's face.

Preferably the seal includes a thick region extending around a perimeter portion of the seal, the perimeter portion surrounding a seal opening.

Preferably the perimeter portion has a perimeter edge defming the seal opening, and a bead is formed at the perimeter edge on an inside surface of the seal.

Preferably the bead is about 0.5mm diameter cross section.

The present invention further provides a patient interface for use as part of an apparatus for supplying a flow of respiratory gases to a user comprising:
headgear,
a mask assembly as outlined in any one of the preceding statements or accompanying claims and including at least one fitting adapted to attach said mask assembly to said headgear.

The present invention is suited for use with an apparatus for supplying a flow of respiratory gases to a user comprising:
a blower unit adapted to provide a stream of pressurised gases,
a humidifier unit adapted to receive said stream of pressurised gases and heat and humidify said stream of gases,
a patient interface,
a conduit connecting said patient interface and said humidifier unit so that said patient interface receives said stream of heated and humidified gases, said patient interface including a mask assembly as outlined in any one of the preceding statements or accompanying claims.

In another embodiment, the present invention provides
a seal having a first side and a second side, the first side of said seal being shaped to approximately match the contours of a user's face and in use substantially seal against a user's face, said second side adapted for direct or indirect attachment to the body, and a wall portion extending around the periphery of the seal between the first and second sides, and the wall portion comprising a circumferential thin portion.

Preferably the circumferential thin portion extends continuously around the full perimeter of the seal.

Preferably the circumferential thin portion extends around only a portion of the perimeter of the seal.

Preferably the circumferential thin portion is formed at an interior surface of the seal wall.

Preferably the circumferential thin portion has an approximately constant width around the perimeter of the seal.

Preferably the circumferential thin portion comprises wide portions and narrow portions.

Preferably the circumferential thin portion may be wider in the nasal bridge region, around the perimeter of the seal or a portion of the perimeter of the seal.

Preferably the circumferential thin portion varies at a repeating pitch in the chin and lower check regions of the seal.

Preferably the circumferential thin portion comprises one or more thin portions extending in a circumferential direction around the seal.

Preferably the circumferential thin portion is in the nasal bridge region only, or chin region only, or in the nasal bridge and chin regions only.

The term "comprising" as used in this specification means "consisting at least in part of'. Related terms such as "comprise" and "comprised" are to be interpreted in the same manner.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting

The invention consists in the foregoing and also envisages constructions of which the following gives examples.

### BRIEF DESCRIPTION OF THE FIGURES

Preferred forms of the present invention will be described with reference to the accompanying drawings.
**Figure 1** is a block diagram of a system for providing a heated humidified gases stream to a user such as a continuous positive airway pressure system as might be used in conjunction with the patient interface of the present invention.
**Figure 2** is a perspective view of a first preferred embodiment of a patient interface of the present invention.
**Figure 3** is an exploded view of the patient interface of Figure 2, the patient interface comprising headgear, a mask seal assembly, a mask cushion and a mask body.
**Figure 4a** is a perspective view of the mask seal assembly of the patient interface of Figure 2 and Figure 3, the mask seal assembly comprising a seal and a seal clip.
**Figure 4b** is a perspective view of the seal clip of the mask seal assembly of Figure 4a.
**Figure 5** is a perspective view showing the mask body and the mask seal assembly, with the mask seal assembly removed from the mask body.
**Figure 6** is a sectional view on line X-X of the patient interface of Figure 2.
**Figure 7a** is a part sectional view on line X-X showing a preferred form of the interface arrangement between the mask body and the mask seal assembly of the patient interface of Figure 2.
**Figure 7b** is a part sectional view showing a first alternative form of the interface arrangement between the mask body and the mask seal assembly of the present invention.
**Figure 7c** is a part sectional view showing a second alternative form of the interface arrangement between the mask body and the mask seal assembly of the present invention.
**Figure 7d** is a part sectional view showing a third alternative form of the interface arrangement between the mask body and the mask seal assembly of the present invention.
**Figure 7e** is a part sectional view showing a forth alternative form of the interface arrangement between the mask body and the mask seal assembly of the present invention,
**Figures 8a and 8b** are perspective views of the mask cushion of the patient interface of the present invention.
**Figures 9a to 9c** illustrate alternative arrangements for providing vent paths between the mask seal assembly and the mask body of the patient interface of the present invention.
**Figure 10** is a side view of the mask body and the mask seal assembly of the present invention.
**Figure 11** is a part sectional view from the side showing the clip and seal arrangement of the mask seal assembly of the present invention.
**Figure 12a and Figure 12b** are part sectional views from the side of a preferred form of connector for connecting a conduit to the mask assembly of the present invention.
**Figures 13a** **and 13aa** show a perspective view and a rear view of a first alternative form of connector for connecting a conduit to the mask assembly of the present invention, the connector incorporating radial baffles extending from the circumferential edge of the connector partway towards the centre.
**Figures 13b** **and 13bb** show a perspective view and a rear view of a second alternative form of connector for connecting a conduit to the mask assembly of the present invention, the connector incorporating a single wave like baffle.
**Figures 13c** **and 13cc** show a perspective view and a rear view of a third alternative form of connector for connecting a conduit to the mask assembly of the present invention, the connector incorporating a single circular baffle centrally located within the connector.
**Figures 13d** **and 13dd** show a cutaway side view and a rear view of a fourth alternative embodiment of connector for connecting a conduit to the mask assembly, the connector incorporating two radially aligned baffles.
**Figure 14** is a part sectional view of the mask seal assembly showing the seal in an un-pressurised state and a pressurised elastically stretched state.
**Figures 15A to 15F** show a side view of various mask seal assemblies each comprising a circumferential thin portion.
**Figure 16** is a cross sectional view of a seal assembly comprising a circumferential thin portion.

### DESCRIPTION OF THE PREFERRED EMBODIMENTS OF THE INVENTION

The patient interface of the present invention is described below with reference to use as part of a system for providing humidified gases to a user for CPAP therapy. The patient interface of the present invention can be used as part of any suitable system that provides a pressurised gases stream to a patient in use. It is particularly suited for use with a system that provides a heated, humidified gases stream to a patient or user.

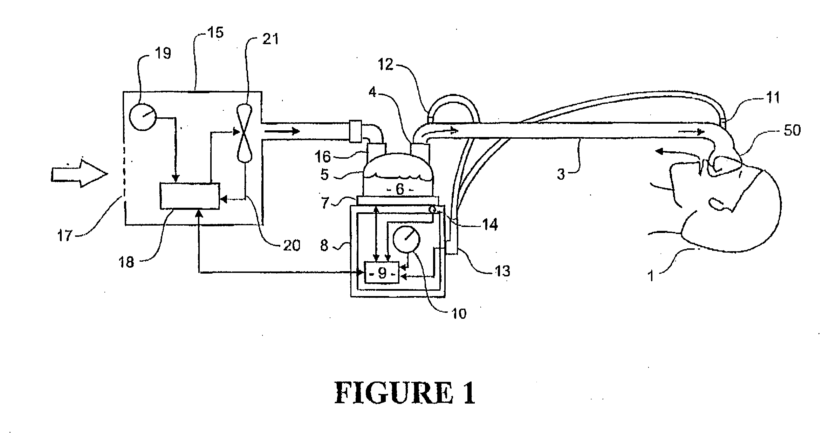

A typical humidified Continuous Positive Airway Pressure (CPAP) system is shown in Figure 1. A pressurised gases stream is provided through the system by a gases supply or blower 15. The pressurised gases stream from the blower 15 is provided to a humidification chamber 5, entering the humidification chamber 5 through an inlet 16. The humidification chamber 5 contains a volume of water 6. The humidification chamber 5 is preferably formed from a plastics material and preferably has a highly heat conductive base (for example an aluminium base) that is in direct contact with a heater plate 7. The heater plate 7 is provided on a humidifier base unit 8. In the system shown in Figure 1, the humidifier base unit 8 and the blower 15 are separate items, but the present invention could also be used with a single, integrated blower/humidifier unit.

A humidified gases transportation pathway or inspiratory conduit 3 is shown connected to an outlet 4 of the humidification chamber 5. The inspiratory conduit 3 may contain heating means or heater wires (not shown) that heat the walls of the conduit to reduce condensation of humidified gases within the conduit 3 in use. A patient interface 50 is connected to the patient end of the conduit 3 so that a stream of heated, humidified air is provided to the patient via the patient interface 50 in use. The preferred form of the patient interface 50 shall be described in detail below.

Either or both of the humidifier base unit 8 and the blower unit 15 are provided with a controller. An electronic controller 9 is shown in the humidifier base unit 8. The controller 9 may comprise a microprocessor based controller executing computer software commands stored in associated memory.

The controller 9 preferably receives input from a user input device such as a dial 10 through which a user of the device may, for example, set a predetermined required value of humidity or temperature (or both) of the gases supplied to patient 1. The controller 9 may also receive input from other sources, for example readings from temperature sensors and flow velocity sensors such as sensors 11, 12 located in the gases stream, and from other sensors such as a heater plate temperature sensor 14. In response to the user set humidity or temperature value input via the dial 10 and the other inputs from e.g. the sensors, the controller 9 determines when (or to what level) to energise the heater plate 7 to heat the water 6 within the humidification chamber 5. As the volume of the water 6 within the humidification chamber 5 is heated, water vapour begins to fill the volume of the chamber above the water's surface and the heated, humidified air is passed out of the humidification chamber 5 via outlet 4.

The blower 15 of the preferred embodiment is provided with a variable pressure regulator. In the most preferred embodiment this is a variable speed fan 20 that draws air or other gases into the blower 15 through a blower inlet 17. In the preferred embodiment, the speed of the variable speed fan 20 is controlled by an electronic controller 18. Alternatively, the function of the controller 18 may be carried out by the controller 9 (or vice-versa). The speed of the fan 20 is adjusted in response to inputs from the controller 18 or controller 9. The predetermined or required user value (preset value) of pressure or fan speed is set by a user or health profession via dial 19, which sends a signal to controller 18 or controller 9, which adjusts the fan speed accordingly.

The patient interface 50 of the present invention is shown in Figure 1 as a nasal mask which covers the user's nose. However the present invention may be incorporated into any mask assembly that seals against a portion of the user's face. For example, the mask assembly could be a full face mask that seals against the user's face around both the user's nose and mouth.

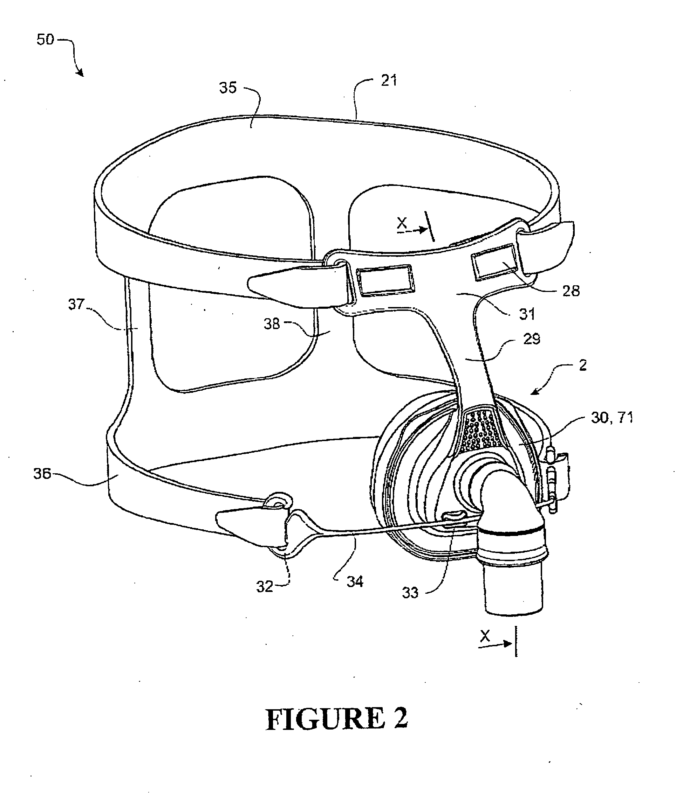

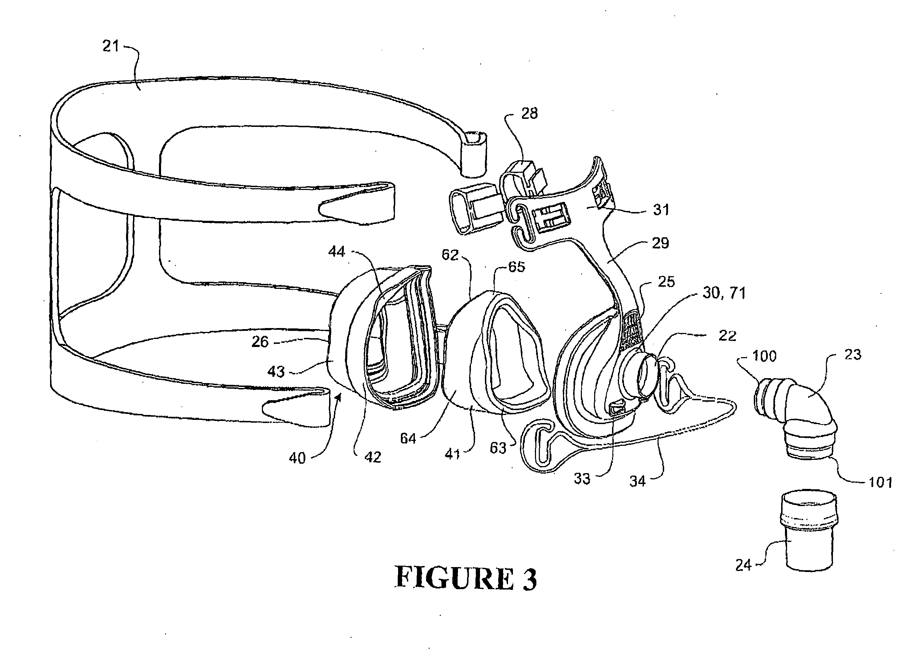

Figure 2 and Figure 3 show details of the patient interface 50 of the preferred form of the present invention. The patient interface 50 includes a mask assembly 2 and headgear 21.

### Headgear

Headgear 21 is worn by the user in use, and holds the mask assembly 2 in the required position on the user's face. The headgear 21 in the preferred form is comprised of headgear straps 35, 36, 37, 38, and an elongate glider member 34. The elongate glider member extends across the front of the mask assembly 2 and attaches to the mask body 30 of the mask assembly 2 via at least one clip type fitting 33. The elongate member 34 may slide, or glide, within the clip type fitting 33 so that the mask assembly 2 may move laterally with respect to the head gear. This arrangement for attaching the headgear to the mask assembly is the preferred arrangement. Other arrangements may also be used with the mask assembly of the present invention, such as a fixed or static arrangement.

Each end of the elongate glider member may include a hook 32 to which each end of headgear strap 35 may releasably attach. Alternatively, headgear strap 35 may attach directly to the mask body 30.

In the preferred form, the mask assembly 2 includes a forehead support 31. The forehead support is attached to the mask body 30 by a vertical support member 29, the forehead support and vertical support member together forming a T shape. The forehead support 31, vertical support member 29 and mask body 30 may be integrally formed as a single piece.

Alternatively, the forehead support may be adjustable in a vertical direction, or may be pivotably attached to the mask body 30.

One end of headgear strap 36 may attach to a first end of the forehead support, and the opposite end of headgear strap 36 may attach to a second end of the forehead support 31. The forehead support may include at least one support pad or cushion 28. In use, the forehead support locates against the user's forehead and provides stability to the nasal interface 50 when fitted to the user.

Headgear straps 37 and 38 extend substantially vertically between headgear straps 35 and 36. Headgear straps 37 and 38 provide support to straps 35 and 36, positioning straps 35 and 36 a set distance apart around the user's head.

The headgear straps are preferably made from a laminated sheet of open cell foam sandwiched between two sheets of textile fabric.

It should be noted that many equivalent forms of head gear known in the art may be suitable for use with the mask assembly 2 of the present invention. What has been described above is the preferred form for use with the mask assembly 2 of the present invention.

### Mask Body

The mask assembly 2 of the preferred embodiment of the present invention comprises a mask body 30 and a mask seal assembly 40.

The mask body 30 provides the overall structural support for the mask assembly, and provides at least one clip type fitting for attaching the mask assembly 2 to the headgear 21.

A rear side of the mask body 30 interfaces to the seal assembly 40, the seal assembly 40 providing a sealing interface against a user's face in use. The rear side of the mask body 30 is the side that faces towards a user, when the user has the mask assembly 2 in place on their face. The mask body 30 has an inlet for receiving a flow of respiratory gases. The mask body forms an internal cavity to which respiratory gases are supplied via the inlet from the CPAP system. The inlet comprises a tubular projection 22 extending from a front side 71 of the mask body 30. The front side 71 of the mask body 30 is the side that faces away from a user, when the user has the mask assembly 2 in place on their face.

### Connector

A first end of connector 23 connects to the mask body tubular projection 22. The interface between the tubular projection 22 and the connector 23 preferably allows the connector 23 to swivel with respect to the mask body 30.

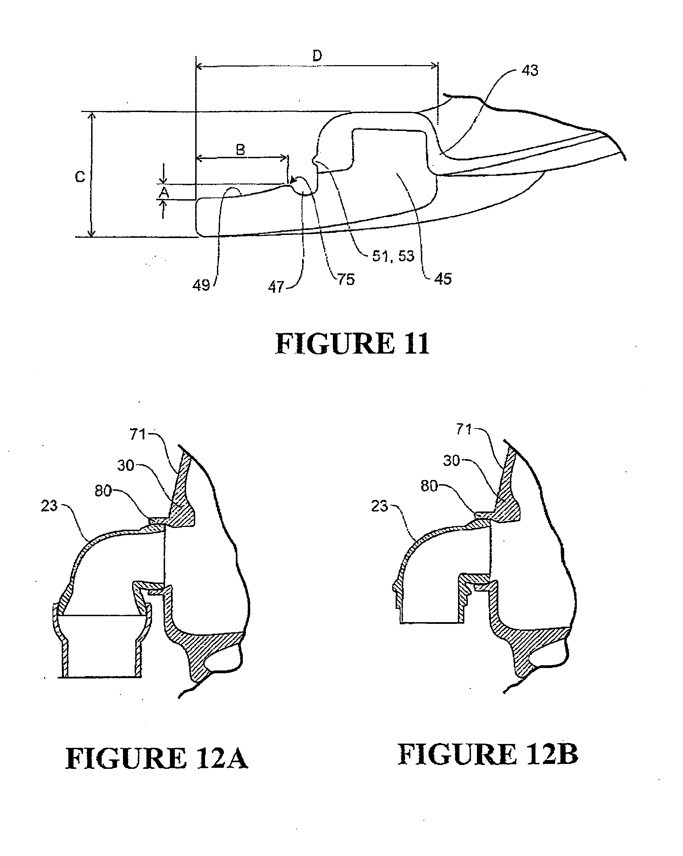

Alternatively, as shown in Figures 12a and 12b, the inlet may comprise a semi-tubular projection 80 extending from the front side 71 of the mask body. The semi-tubular projection forms a socket for receiving a correspondingly shaped first end of the connector 23. In use, the first end of the connector and the semi-tubular projection form a ball joint allowing the connector 23 to swivel and pivot within the socket, relative to the mask body 30. In alternative embodiments the mask body 30 may not include a projection, and the inner surface of the mask body 30 may be curved to form a socket for receiving the connector. In other alternative forms, other types of connection may be utilised between the mask body and the connector, such as a flexible piece of silicone, or other appropriate connection mechanism.

The connector 23 is preferably an elbow connector having a first leg, a bend and a second leg. The bend is preferably a substantially 90degree bend.

A second end of the connector connects to the inspiratory conduit 3, either directly to the conduit 3, or via a second connector 24. The interface between the elbow connector 23 and the conduit 3 or the second connector 24 preferably allows the conduit 3 to swivel with respect to the connector 23.

Alternatively, the interface between the second end of the connector and the conduit 3 may comprise a ball joint that allows the second end of the connector to swivel and pivot relative to the conduit, as shown in Figure 12A.

Preferably the connection between the mask body 30 and the conduit 3 can be flexed or rotated to allow for the conduit to be moved without causing the dislodgement of the mask assembly 2 from the user's face. This is a preferred feature of the present invention, but is not essential to the present invention.

As best shown in Figure 3, the preferred form of elbow connector 23 is a substantially right angle connector, with the tubular inlet projection 22 aligned substantially perpendicular to the front of the mask body 30. Other alternative arrangements may be incorporated in the mask assembly of the present invention. For example, the connector may be a straight connector, with any necessary bend accommodated by the conduit 3. Alternatively, the tubular inlet projection may extend from the front of the mask body at an angle, for example angling downwards, with the connector having a corresponding obtuse angle.

Preferably the connector 23 may be easily removed from the mask body by pulling the connector 23 away from the mask body 30.

### Reducing diameter connector

As shown in Figure 3, in the preferred form, the first end 100 of the elbow connector 23, interfacing with the mask body 30, is smaller in diameter than the second end 101 of the elbow connector. Preferably the diameter of the connector 23 reduces around the length of the bend in the connector 23, from the full diameter at the second or outer end, to the reduced diameter at the first or inner end. As an example, the diameter of the inspiratory conduit commonly used in the art is approximately 25mm. Prior art masks continue this full diameter through to the mask body interface. In the preferred form of the present invention, the diameter of the connector 23 at the mask body interface is reduced to approximately 20mm.

It is desirable to have a mask assembly that is as small and lightweight as possible, while at the same time providing an effective seal against a user's face. The reducing diameter of the connector 23 helps to make the mask assembly lightweight and small. The reducing diameter of the connector 23 helps to visually give the mask assembly a small, compact look.

One problem with a connector 23 having a reducing diameter connector is that air flow through the connector can become unacceptably noisy. Noise is created as the air flow passes through the reducing cross section of the connector. It is thought that this noise is created by an increase in the speed and turbulence of the flow of inspiratory gases through the reduced cross section portion 102 of the connector 23. The additional noise level is clearly undesirable as it can disturb the user's sleep, or the sleep of a user's partner.

To overcome the problem of increased noise levels, the connector 23 of the preferred embodiment of the present invention includes at least one baffle 103 located in the reduced cross section portion of connector 23.

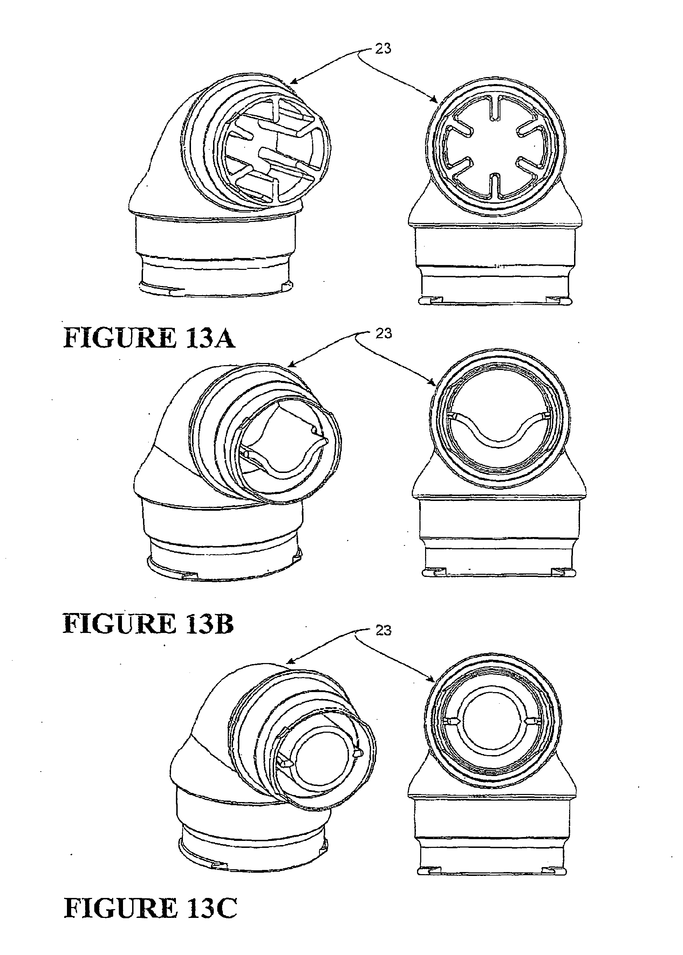

Figures 13a and 13aa show a first alternative form of the connector 23, including six equally spaced baffles 103, each baffle extending from a first end 100 of the reduced cross section portion 102 of connector 23 to a second end 104 of the reduced cross section portion of connector 23. The introduction of the baffles significantly reduces the noise create by the reduced cross section portion.

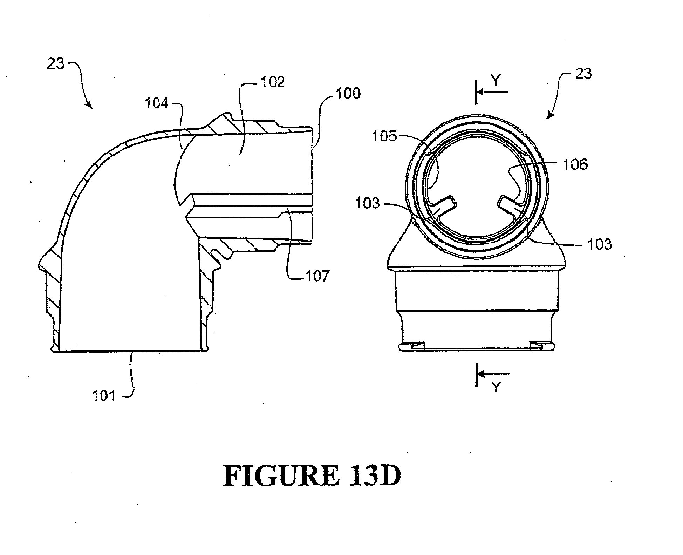

A downside to introducing the baffles is a higher pressure drop through the connector 23. It has been determined by the inventors following extensive research that a significant reduction in noise level is achieved by incorporating two baffles only, the baffles located at the third and fifth positions when considering six equally spaced positions spaced from top-dead-centre around the circumference of the reduced cross section portion of connector 23, top-dead-centre being position 1, with the second leg of the elbow at the downwards fourth, or 6 o'clock, position. This arrangement is shown as a fourth alternative embodiment in Figures 13d and 13dd.

The noise reduction achieved by incorporation of two baffles only in the positions described is similar to the noise reduction achieved with incorporation of all six baffles. The introduction of two baffles in the third and fifth positions results in a significant reduction in noise levels, a similar result to the introduction of six baffles as shown in Figures 13a and 13aa. However, the pressure drop in the flow of gases through the connector 23 due to the incorporation of two baffles is lower than the pressure drop with six baffles. The most preferred form of baffle arrangement is therefore to include two baffles, located at the positions described, resulting in an acceptable compromise between acceptable noise levels and acceptable resistance to flow through the connector 23. The fourth alternative embodiment, which shows the most preferred form of baffle arrangement is shown in Figures 13d and 13dd. The baffles are preferably aligned radially within the reduced cross section portion of connector 23.

In the preferred embodiment the baffles connect to the circumferential wall 105 of connector 23. The baffles extend along the wall 105 substantially parallel to a longitudinal axis of the reduced portion 102. Preferably the baffles extend from the second end 104 of the reduced diameter portion to the first end 100 of the reduced portion. Alternatively, the baffles may extend part way along the reduced diameter portion. Preferably the baffles extend along the full length of the reduced diameter portion 102 and connect along the wall 105, along the full length of the baffle except for an end portion 107 near the first end 100 of the connector 23, the end portion 107 of each baffle 103 being detached from the side wall 105. The end portion 107 of each baffle 103 is not connected to the wall 105 of the connector 23 so that additional strength provided by the baffles is not applied to the connector wall at the first end 100. This allows the wall 105 at the first end 100 to deflect more easily when connecting connector 23 to the mask body 30.

The baffles are around 1.5mm thick and around 3mm in radial height from the side wall 105. The thickness of the baffle is not overly important for achieving a reduction in noise level. The thickness dimension is chosen to allow ease of manufacturing. The connector 23 is typically manufactured by plastic injection moulding. Other injection moulding techniques may be incorporated for ease of manufacture, such as drafting the internal bore of the connector and drafting the baffles. Preferably the sides 106 of the baffles 103 have a draft of around 0.5°. The edge 107 of the baffles 103 have the same draft as the internal diameter of the reduced diameter portion 102, that is, a draft of around 1° to 1.5°.

Further research found that the radial height of the baffles had a significant effect on the reduction in noise levels. A significant reduction in noise level was obtained with a baffle radial height of 3mm. There was a large improvement in noise reduction by increasing the radial height from 2mm to 3mm. However, the improvement achieved by increasing the radial height from 3mm to 4mm was not as significant.

In an alternative embodiment the baffles 103 may be connected to form a continuous bridge across the cross section of the reduced diameter portion 102. However, for performance and ease of manufacture this is not a preferred embodiment.

Alternative baffle arrangements have also been found to be useful in reducing noise levels of gases flow through connector 23. Figures 13b and 13bb show a single baffle that extends across the reduced cross sectional area of the connector, the baffle being curved to extend towards the side nearest the elbow bend of connector 23, that is, the side opposite top-dead-centre with the second leg in the downward 6o'clock position. Figures 13c and 13cc show a single circular baffle located centrally within the reduced cross section portion of connector 23, the circular baffle being supported by extensions at the six and three o'clock positions connecting between the outer wall of the single circular baffle and the inner wall of the connector. However, based on testing carried out by the inventors, these alternative embodiments are not the most preferred forms.

The use of baffles may also be helpful in reducing noise levels in an elbow connector with equal diameter legs, as turbulence in the gases flow is caused by flow around the bend of the elbow.

Alternatively, the use of baffles may also be helpful in reducing noise levels caused by a gases flow through a reducing diameter connector that has a straight through bore.

### Mask seal assembly and mask seal assembly to mask body interface

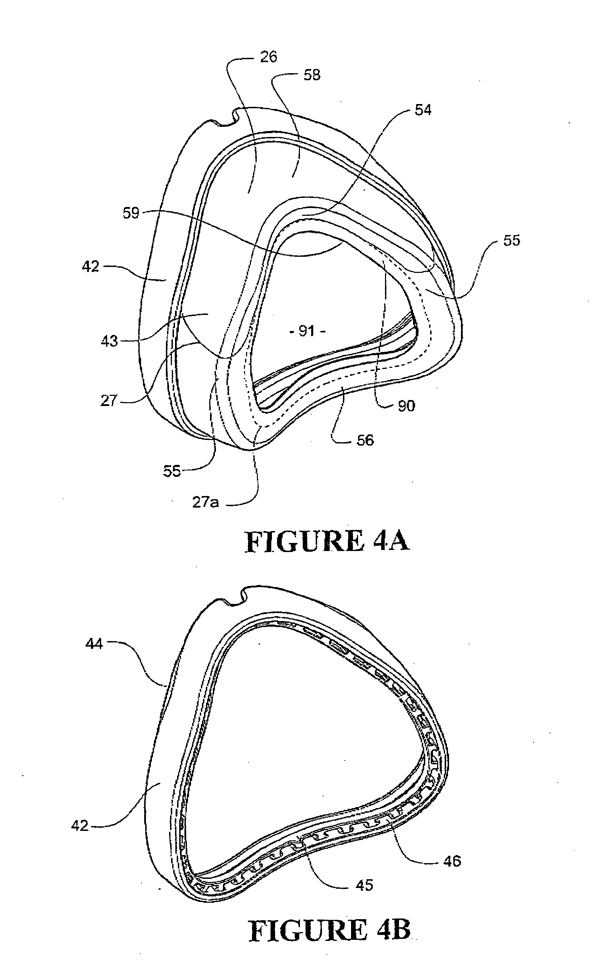

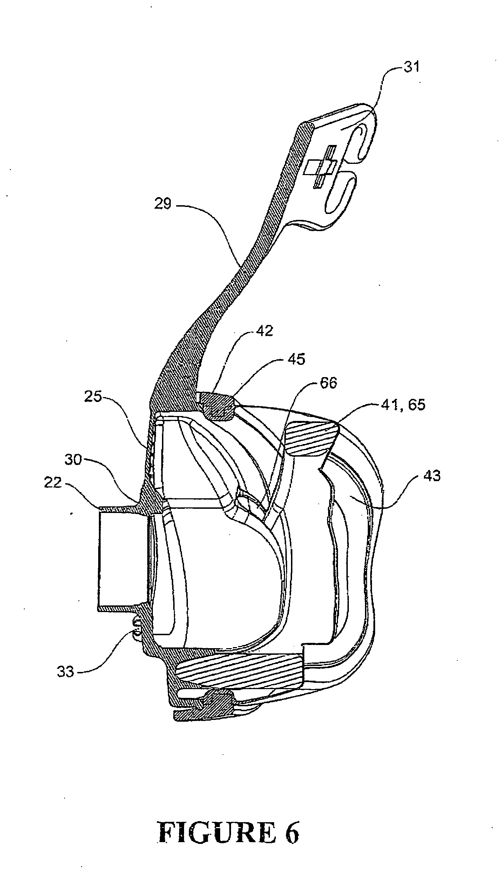

Preferred and alternative forms of the mask body 30 and a mask seal assembly 40 shall now be described with reference to Figures 4 to 11.

In the preferred form, the seal assembly 40 comprises a flexible seal 43 attached to a relatively rigid plastic clip 42, as shown in Figure 4a. Preferably the flexible seal 43 is permanently attached to the plastic clip 42 so that the seal assembly 40 forms a single item of the mask assembly 2.

In the preferred form, the seal 43 is over-moulded to the plastic clip 42. The plastic clip has a series of holes 46 around its perimeter. During manufacture, over moulding of the seal to the clip causes the seal material to flow through the series of holes 46. During manufacture, the seal material is cured. Once cured, the seal 43 is mechanically linked to the plastic clip 42 via holes 46, providing a mechanical joint between the clip and the seal. In the preferred embodiment, the holes 46 are located through a raised ridge 45 running around the inside perimeter of the clip. Preferably the raised ridge 45 and holes 46 are located on a inside surface of the clip, so that the mechanical bond is located on the inside of the clip, with the joint between the clip 42 and the seal 43 visible as a simple butt joint on the outside surface of the seal assembly 40.

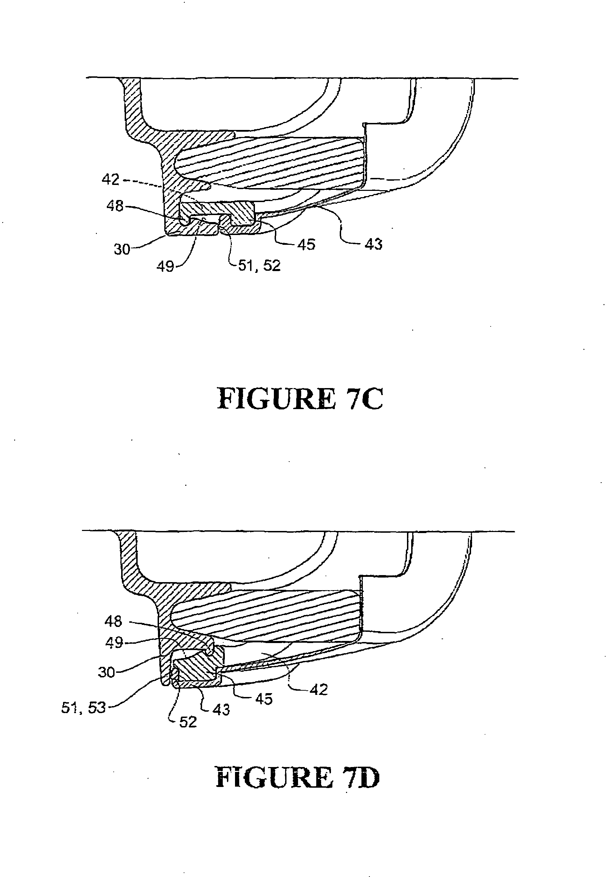

In alternative embodiments, the raised ridge 45 may be located on the outside perimeter of the clip, as shown in Figures 7c and 7d.

Alternatively, the seal 43 may be chemically bonded to the plastic clip. Chemical bonding may be used in combination with the mechanical bond described above.

Alternatively, the seal 43 could be glued to the plastic clip 42 with an appropriate adhesive, or the seal could be mechanically fixed to the clip. For example the seal could be sandwiched between the clip and a flange, with screws or other mechanical fixings securing clip and the flange together, with the seal sandwiched in between. This type of mechanical fixing could be permanent, or the fixings, such as screws, may allow the seal to be dismantled from the plastic clip.

Alternatively, the seal 43 could be assembled to the clip 42 in a press fit arrangement. The press fit may allow the seal 43 to be attached to and detached from the seal clip 42 many times. Alternatively, the press fit may be a permanent connection of the seal 43 to the clip 42.

The clip 42 forms a rigid perimeter for the seal assembly 40, the clip 42 being rigid relative to the seal 43. The clip provides a rigid interface extending the full perimeter of the seal assembly or at least substantially the full perimeter of the seal assembly.

The interface between the rigid clip 42 and the rigid mask body 30 provides a positive engagement between the seal assembly 40 and the mask body 30. This positive engagement provides an improvement over prior art masks that have a flexible seal interfacing directly to a mask body. The face seal assembly of the present invention may be easily and quickly assembled and disassembled to and from the mask body in a single engagement action. Prior art masks with a flexible seal to mask body interface require a comparatively difficult seal to body engagement, as discrete portions of the seal must be fitted one at a time to the mask.

Preferably the seal assembly clip 42 and mask body 30 are made from polycarbonate, nylon, acetyl or other similar rigid plastic.

Alternatively, the seal assembly clip 42 and mask body 30 could be made from a semi-rigid material such as a thermoplastic elastomer, silicone rubber or urethane, or other similar semi-rigid material. Preferably the semi-rigid material has a Shore-A hardness of 60 or higher.

The clip 42 is shaped to match the shape of the back perimeter region 52 of the mask body 30. The clip 42 may only be attached to the mask body in a single orientation. It is therefore not possible to attach the seal assembly 40 to the mask body 30 incorrectly.

The clip 42 attaches to the mask body in a 'clip' or 'snap' type engagement. A series of bumps 48, or raised portions, on the mask body 30 interact with corresponding recesses 47 on the clip 42, to hold the clip 42 in place on the body 30. For example, for a mask that has a substantially equilateral perimeter with three sides, three recesses 47 may be located on the inside surface of the clip, the recesses being spaced apart, one recess being located on each side of the equilateral perimeter. The mask body, being correspondingly shaped to match the seal assembly clip, has three corresponding bumps 48. As the clip 42 attaches to the mask body, interference between the clip and each mask body bump 48 causes the clip or the mask body, or both, to deflect to a deflected condition until each bump 48 reaches a corresponding recess 47. Once the clip has been fully engaged with the body, each bump 48 locates within a corresponding recess 47, and the clip or body, or both un-deflect from the deflected condition to an un-deflected or partially deflected condition, the parts being clipped or snapped together in a fully engaged position. Preferably the clip or the mask body or both remain in a slightly deflected condition when fully engaged together.

The recesses 47 may be formed by having a second series of bumps on the clip, each recess effectively created by a rear shoulder of a bump. In this arrangement, interference between bumps on the clip and bumps on the mask body occurs until the clip bumps and the mask body bumps pass to sit adjacent one other in an engaged position

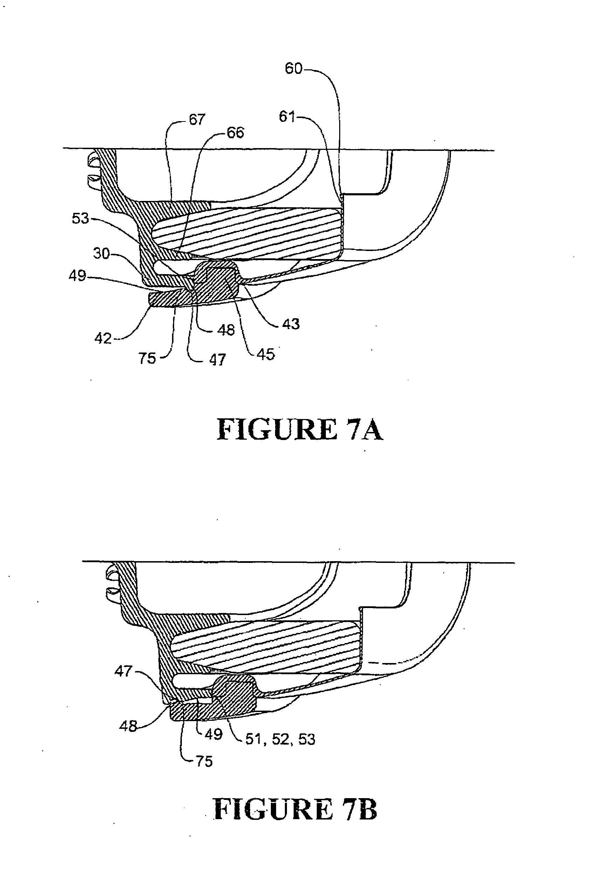

As best shown in Figures 7a and 7d, the clip 42 preferably has a relatively long lead in, or ramped profile 49, leading to the clip recess 47. This lead in section preferably extends the full inside perimeter length or substantially the full inside perimeter length of the clip 42. Preferably the lead-in section extends at least 50% of the full perimeter length of the clip.

Preferably each recess 47 is located immediately behind the lead-in section. The lead-in section assists with the attachment of the clip to the mask body. The clip 42 or mask body 30, or both, are gradually deflected over the length of the lead-in section until the apex 75 of the lead-in section and each mask body bump 48 pass each other. Once the bumps 48 have passed over the lead-in section, the bumps 48 locate within each corresponding recess 47, such that there is little or no interference between the two parts 30 and 42. The two parts un-deflect in a relatively sudden snap action compared to the gradual deflection caused by the lead in section 49 during engagement. This arrangement provides a positive, single engagement action. The positive single engagement action provides the user with assurance that the seal assembly has been correctly fitted to the mask body. The clipping arrangement preferably generates a clipping sound as the bumps 48 located into the recesses 47. The clipping sound provides reassurance to the user that the clip has been correctly fitted to the mask body. The seal assembly 40 may be attached to and detached from the mask body 30 many times.

Alternatively, the lead in section, or ramp, described above may be provided on the mask body, as shown in the alternative embodiments of Figures 7b and 7c.

The lead in or ramped section 49 has a length to height ratio of approximately 3 to 7. In the preferred embodiment the lead in section has a length to height ratio of around 5. That is, for a ramp length of around 5mm, the ramp height is around 1mm. The lead in section ratio is best identified in Figure 11, where the ramp length B is around five times the ramp height A.

The ramp section results in a seal clip cross section with a relatively high width to height ratio. The cross section of the preferred embodiment of the seal clip has a width to height ratio of around 2. Once the seal 43 is attached to the clip 42, the cross section of the seal assembly 40 at the seal clip has a width to height ratio of around 1.5 - 2, with the shorter dimension transverse to the direction in which the clip is engaged to the mask body. Preferably this ratio is around 1.8.

In the preferred embodiment of Figure 7a, and in the alternative embodiments of Figures 7b and 7d, the clip 42 interfaces with the mask body such that the clip engages to an outwardly facing surface of the mask body. However, in the alternative embodiment of Figure 7c, the clip 42 interfaces to an internal or inwardly facing surface of the mask body.

A series of bumps 48 and recesses 47 around the perimeter of the mask body and clip have been described above. The bumps and recesses are located so that the seal assembly may be disengaged from the mask body by squeezing the sides or opposite perimeter portions of the clip, to deflect the clip and disengage the bumps 48 from the recesses 47. To disengage the clip from the mask body, opposite perimeter portions of the clip are squeezed at positions where bumps 48 are absent, allowing the clip to deflect, to 'pop' the bumps out of the corresponding recesses. Given the ratio of the seal assembly cross section at the clip, as described above, the clip is relatively thin transverse to the direction in which the clip is engaged to the mask body. The thin clip is relatively easy to deflect by squeezing the opposite perimeter portions in a direction transverse to the direction in which the clip engages to the mask body, to easily deflect the clip from the mask body.

Alternatively, a continuous bump extending fully around the mask perimeter may be utilised to provide the snap interface between the seal assembly 40 and the mask body 30. In this configuration, the bump may not have a constant height all the way around the mask perimeter. Having a continuous bump with different heights may allow the clip to be disengaged from the mask body by squeezing the sides or opposite perimeter portions of the clip, to deflect the apex 75 of the lead in section 49 of the clip 42 past a corresponding higher portion of the continuous bump.

Alternatively the clip 42 may have a continuous recess passing around the full perimeter or substantially the full perimeter of the clip, the recess being located immediately behind the lead-in section 49, the mask body bump 48 passing over the lead in section before clipping into a portion of the continuous recess.

In alternative embodiments such as the embodiments shown in Figures 7b and 7c, the recesses 47 may be provided in the mask body 30, and the corresponding bumps may be formed on the mask clip 42.

The face seal assembly 40 may include at least one wing portion 44 to assist a user to disengage the face seal assembly from the mask body. The wing portions 44 provide a gripping flange to pull the clip 42 away from the mask body 30.

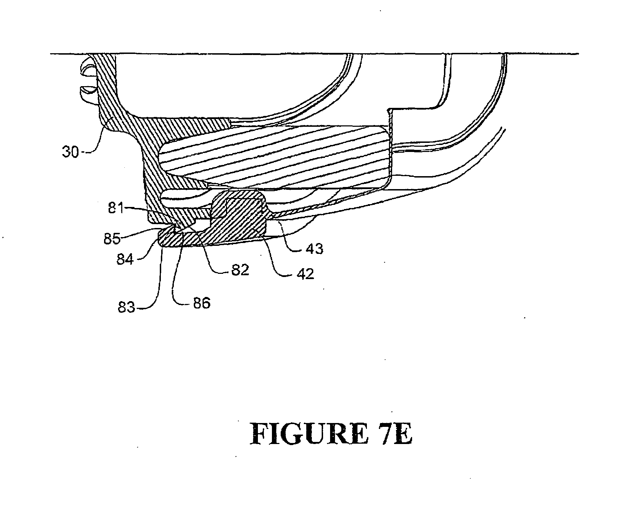

In a further alternative embodiment, the clip may be clipped to the mask body in a permanent engagement. With reference to Figure 7e, the mask body 30 has a series of protrusions 81. Each protrusion has a ramped surface 82 and an abutment surface 83. For each body protrusion 81, the mask clip 42 has a corresponding protrusion 84, each mask clip protrusion having a ramp surface 85 and an abutment surface 86. Alternatively, a single continuous protrusion 81 may pass around the perimeter of the mask, with a corresponding continuous protrusion 84 passing around the perimeter of the clip. During an assembly process, the seal assembly clip 42 is interfaced to the mask body in a pressing operation, the mask body and mask clip are pressed together. The mask body, or the mask clip, or both deflect as the ramp surfaces 82 and 85 contact and slide past one another. Once each mask body protrusion 81 and mask clip protrusion 84 pass each other, mask body 30 or clip 42, or both, return to an un-deflected or partially deflected condition, and mask body protrusion abutment face 83 abuts against clip protrusion abutment face 86, retaining mask body 30 and clip 42 together. The abutment faces 83 and 86 are aligned substantially laterally relative to the direction in which the clip and body are pressed together. With the abutment faces arranged laterally, or substantially perpendicular to the pressing direction, the clip 42 may not be removed from the mask body easily, creating a substantially permanent joint between the clip and the body.

This alternative embodiment is useful for disposable masks that are disposed of after a single use. Such an embodiment is useful in simplifying the manufacturing process. During manufacture, moulding the seal 43 to the seal clip 42 may be easier than moulding the seal 43 directly to the mask body 30. Once the seal is moulded or attached to the seal clip as in any of the embodiments described above, the subsequent operation of pressing the clip 42 and mask body 43 together is a relatively simple assembly operation.

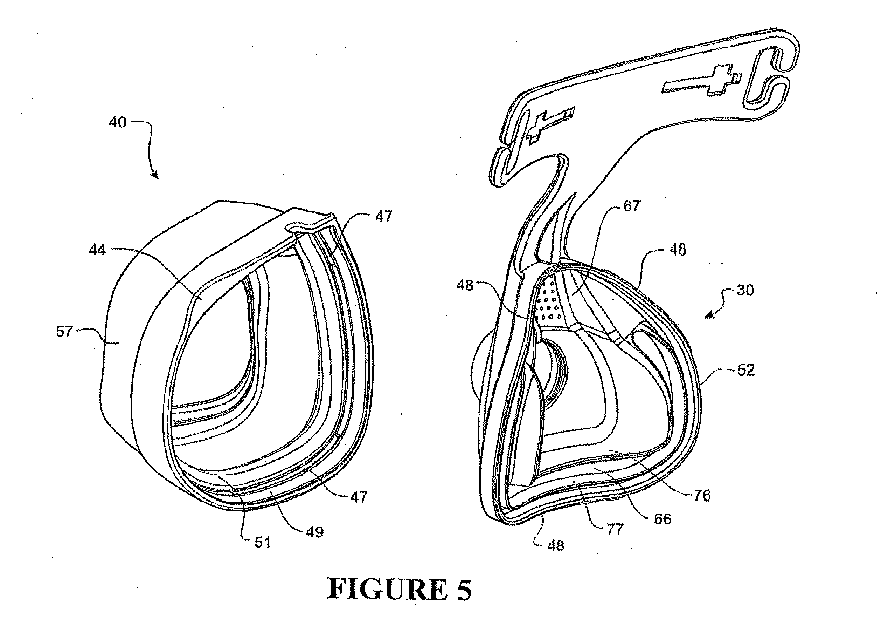

As best shown in Figure 5 and Figure 7a, the seal preferably has a bearing surface 51 extending around the perimeter of the clip. The seal bearing surface 51 faces a corresponding bearing surface 52 on the mask body. The seal bearing surface and the mask bearing surface are in contact when the seal assembly is attached to the mask body. When the seal assembly 40 is attached to the mask body 30, the seal 43 at the bearing interface is compressed so that a sealing interface is formed between the seal assembly and the mask body. Preferably, the clip has a raised ridge 45 running around the inside perimeter of the clip. The seal 43 is compressed by being squashed between the raised ridge 45 and the mask body bearing surface 52 when the seal assembly 40 is attached to the mask body 30.

To assist with creating a good seal between the seal assembly and the mask body, a continuous rim 53 may be provided on the seal bearing surface 51. The rim provides a small contact area in contact with the mask body bearing surface 52. The small contact area allows a relatively high compression of the rim, and therefore effective seal, for a relatively small seal assembly to mask body engagement force.

### Profiled seal clip

The seal 43 is shaped to approximately match the facial contours of a user's face. For the preferred form of the nasal mask shown in the accompanying Figures, the face seal is contoured to approximately match the facial contours of a user around the user's nose, from the bridge of the nose, continuing down the cheek regions adjacent each side of the user's nose and across the user's philtrum area. In particular, there is an indented section 54 intended to fit over the bridge of the patient's nose, a cheek contour 55 on each side to follow the cartilage extending from the middle of the user's nose, and an indented section 56 to seal around the philtrum area of the user.

The present shape of the seal is chosen to 'approximately match' the facial contours ofa range of users. To approximately match the contours of a user's face, the seal is contoured in three dimensions, that is, shaped to fit around a user's nose, and contoured in a direction substantially normal to a user's face, as described above and shown in the accompanying figures.

Similarly, if the invention was applied to a full face mask covering a user's nose and mouth, the face seal would be shaped to approximate the facial contours of the user's chin and wider check regions.

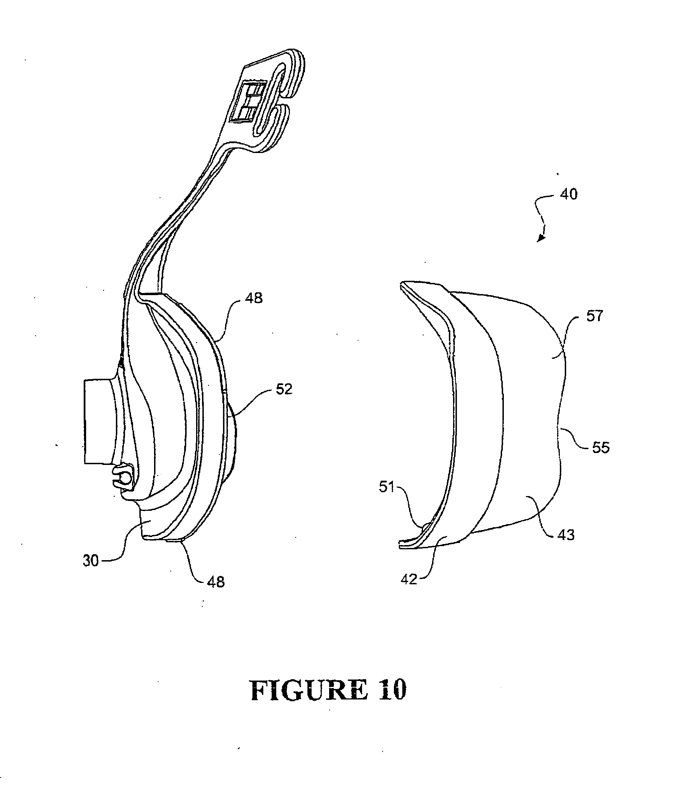

As best shown in Figure 10, the seal assembly clip 42 and the rear portion, or back edge 52 of the mask body are shaped to generally follow the contours of the user's face. Shaped to 'generally follow', means that the contoured shape of the portion of the seal in contact with a user's face is generally replicated in the shape of the clip. The profile of the clip and the profile of the seal in contact with the user's face are therefore similar. The main difference in the shape of the clip and the shape of the portion of the seal in contact with the user's face is that the shape of the clip does not include the indent 55 in the cheek contour portion of the seal, the shape of the clip being flatter in this region. The profiled clip allows the walls 57 of seal assembly 40 to have a generally constant depth around the circumference of the seal 43. Preferably the wall depth varies by less than around one third of the deepest wall depth. A generally constant wall depth helps the seal to apply even pressure to the face around the circumference of the seal. The profiled seal clip 42 and mask body 30 also helps to minimise the size of the mask assembly. Minimising the size of the mask is an important feature for a comfortably fitting mask. A small size provides a lightweight mask that is more comfortable to wear and less obstructive to the user.

A profiled seal assembly clip 42 and a corresponding profiled mask body 30 provides the additional benefit that a small amount of misalignment may be corrected when fitting the seal assembly to the mask body due to the shape of the parts. For example, the inventors have found that the profiled shape of the clip and mask body, combined with the ramp section 49 that extends the full perimeter length or substantially the full perimeter length of the clip, automatically corrects an angular misalignment of approximately 5 degrees when engaging the clip to the mask body. When pushing the clip and body together, misalignment is corrected as a portion of the mask body contacts the lead in section 49 of clip 42. For example, given a small amount of angular misalignment between the clip and mask body, the initial contact between the clip and the mask body occurs at a first position on one side of the nasal bridge region of the mask body and at a second position on the mask body diametrically opposite the first position. The two positions of the mask body in contact with the clip are angled partially in line with the direction in which the clip is engaged to the mask body. As engagement force is applied to the clip and mask body, the angled shape of the clip and mask body causes the clip and the mask body to slide into alignment until the clip and mask body snap together. In prior art masks with a planar interface between the mask body and seal clip, the interface between the clip and the mask body is transverse to the direction in which the clip engages to the mask body; the seal clip and the mask body must be accurately aligned in order to correctly fit the clip to the body.

### Inflatable seal

Preferably the seal 43 includes a thin region 26. The thin region 26 is a region formed in the seal 43 which is thinner than the remainder of the cross-sectional thickness of the seal 43. In the preferred embodiment the thin region is in the nose bridge region, the extent of which is indicated by line 27 in Figure 4a. The thin bridge region 26 is shown to extend approximately halfway down the sides of the seal from the apex 58. Alternatively, the thin region 26 may extend around the perimeter of the seal 42, as indicated by line 27a in Figure 4a.

In particular, the edge portion 90 must be thicker than region 26 so as to provide strength to prevent the seal losing its shape under typical CPAP pressures. The seal thickness is also thicker at the interface between the seal 43 and the clip 42, to provide strength in the joint between the seal 42 and the clip 42.

For example, the thick portions of the seal may have a thickness of around 0.3 to 0.6mm. The thin region has a thickness that is in the range of around 20 - 80% of the thickness of the rest of the seal, that is, a thickness of around 0.05mm - 0.5mm. Preferably the thin section is ultra thin with a thickness of around 0.05mm - 0.2mm. In the preferred embodiment, the ultra thin region reduces the pressure on a patient's nose in the nasal bridge region, compared to when a seal does not have any reduced thickness section. Furthermore, a thin region 26 in the seal 43 allows for different sized patient's to comfortably use the mask and seal of the present invention.

In use, when a force is placed against the seal 43 the thin region 26 will yield more than the rest of the seal 43. Therefore, this region 26 is more flexible and allows for added patient comfort.

In addition to being more flexible, due to the thin region being so thin, in the preferred embodiment the thin region 26 inflates as the CPAP pressure is applied to the mask.

Prior art masks include seals that inflate under typical CPAP pressures. However, prior art nasal masks inflate to a preformed shape or a displaced preformed shape. The preformed shape is loose when not in use. In use, the loose shape fills with air or gases and is supported in the preformed shape or a displaced preformed shape by the air or gases at positive pressure being supplied by the CPAP system. The prior art mask seal does not inflate to an elastically stretched state significantly larger than the molded or formed shape of the un-pressurized seal. For example, prior art mask seals include a bellows section which is loose when not in use. When in use, the bellows section fills up with air. The seal material of the bellows section is not significantly stretched, as the material thickness of the bellows section does not allow for significant elastic stretch under normal CPAP pressures, the material of the bellows section being too thick.

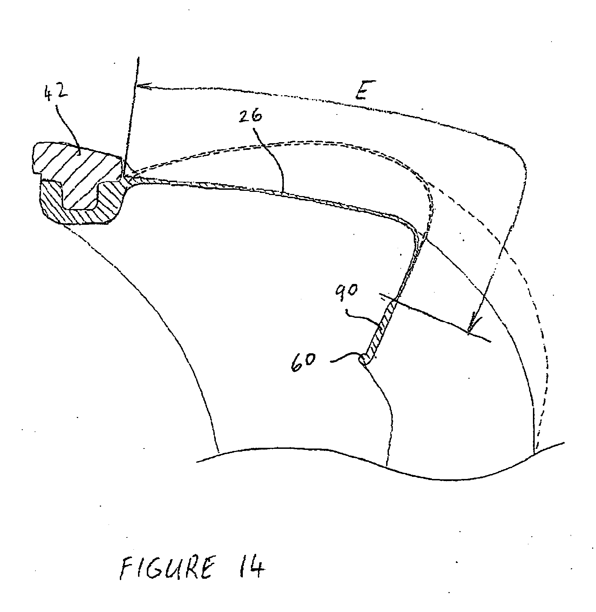

In the preferred embodiment of the present invention, the ultra thin section 26 inflates under CPAP pressure to an elastically stretched state significantly larger than the molded or formed shape of the seal. The ultra thin region provides a seal that remains tight, with no loose sections that may crimp or fold, when un-deflated. Inflation by typical CPAP pressures to a significantly stretched state helps the sealing performance of the mask, as the seal elastically inflates to the facial contours of the user's face and allows for more movement of the mask on the user's face. The ultra thin region 26 inflates to an elastic stretch condition under typical CPAP pressures of 4cm - 25cm H₂O head. The seal of the present invention includes a thin region that inflates under typical CPAP pressures to an elastically stretched condition with strain in the thin region greater than 0.1. That is, the length E of the thin region as shown in Figure 14 increases by at least 10% when pressurized under normal CPAP pressures. In Figure 14, seal 43 drawn in continuous line type shows the thin region 26 un-pressurized. The dashed lines show the seal 43 pressurized, with the thin region 26 pressurized to a significantly stretched condition, with an elastic stretch of approximately 10%. Preferably the thin region inflates under typical CPAP pressures to an elastically stretched condition with strain in the thin region greater than 0.2.

The seal is typically formed during manufacture by injection molding. Such a thin region has been achieved in manufacture by injecting the seal material into a closed seal mold via the thin region 26. Having the injection point within the thin region allows better control of the seal forming process and allows the ultra thin region to be formed successfully. Injection via the thin section is preferred.

The thickness figures provided above are preferable for silicone with a shore A hardness of approximately 40. If an alternative silicone or other rubber material with similar properties to silicone is used, the thickness figures should be adjusted. The thickness figures may be adjusted inversely proportionally with respect to the hardness or elasticity of the material. For example, for a material with a Shore A hardness of 20, the seal thickness may be twice as thick as the figures provided above.

As best shown in Figure 4a, the thin region 26 on the seal 43 preferably does not extend completely to the outer edge 59 of the seal 43, but grows in thickness in region 90. This is because the outer edges of the seal 59 when thicker are less prone to tearing. Also thicker region 90 provides structural support to the seal 43 so that seal 43 maintains its perimeter length in contact with the user's face when inflated, the perimeter length extending around the seal opening 91.

In additional, as shown in Figure 7a, the seal preferably has a bead 60 on the perimeter edge of the seal surrounding the seal opening 91. Preferably this bead is formed at the inside surface 61 of the seal. The diametrical cross-section of the bead will typically be in the range 0.4mm - 1.0mm. Preferably the bead diameter will be about 0.5mm. The bead provides additional strength to the edge of the seal, to add additional strength and support at the perimeter of the seal opening 91 and reduce the likelihood of the seal being torn if the seal is inadvertently caught or snagged by a foreign object.

Alternatively, the seal 43 may be molded without an opening. Opening 91 is then cut out of the molded seal in a cutting operation following the molding process. The opening 91 may be cut with a knife or press in the cutting operation. The cutting operation results in a clean edge which is less susceptible to tearing compared to a molded edge. For a seal manufactured in this way, a bead 60 is less important.

### Mask cushion

The mask assembly preferably includes a mask cushion 41. A prior art mask cushion is described in US 7,308,895.

In the interface 2 of the present invention, the cushion 41 is provided around the periphery of the mask, and is surrounded by the seal assembly 40. When using a thin seal, the cushion 41 provides support to the seal assembly 40 to achieve an effective seal onto the face of the user to prevent leakage.

One end 62 of the mask cushion is shaped to match the shape of the seal in contact with the user's face, and an opposite end 63 is shaped to match the mask body.

As described above, the seal assembly clip 42 and mask body 30 generally follow the contours of the user's face, and therefore provide a generally constant seal depth between the mask body 30 and seal surface in contact with the user's face. The cushion in the preferred embodiment therefore has a wall 64 with a generally constant depth. The constant depth cushion helps to provide an even pressure to the face around the periphery of the mask.

The cushion is constructed of a resilient material, for example polyurethane foam, to distribute the pressure evenly along the seal around the patient's face. In other forms the cushion 41 may be formed of other appropriate material, such as gel, silicone, or other composite materials.

In the preferred embodiment, the mask cushion includes a raised bridge 65 in the nasal bridge region. The raised bridge 65 can also be described as a cut out section made in the cushion, the cut out being on the mask body end 63 of the cushion. As the raised bridge 65 is unsupported by the mask body 30, it is much more flexible and results in less pressure on the nasal bridge of the patient.

In other forms, the cushion may be provided with other contours or cut outs on the mask body end 63 of the cushion, so that in the areas where there are regions cut out, the cushion is more flexible.

Preferably, the extent of the raised bridge portion 65 of the cushion substantially aligns with the extent 27 of the thin bridge section 26 of the seal described above.

The cushion 41 is located around the outer periphery of the mask body, contacting the mask body except for in the raised bridge portion 65 of the cushion. As best shown in Figure 5, the cushion is located in a generally triangular cavity 66, the cavity continuing around the periphery of the body, terminating at each side of the nose bridge region 67 of the mask, where the raised bridge portion 65 of the cushion does not contact the mask body 30. The cavity 66 is generally formed by two spaced apart walls 76 and 77.

The cushion 41 in the preferred embodiment is a separate item, the seal assembly 40 fitting in place over the cushion to hold it in place within the mask assembly 2. Alternatively, the cushion may be permanently or releasable attached to the seal assembly 40 so that the seal, seal clip and cushion may be provided as a single assembly. The cushion may be permanently or releasable attached to the seal 43 or to the seal clip 42. Alternatively, the cushion may be permanently or releasable attached to the mask body 30.

### Exhaust holes

In use the user exhales his or her breath either via an expiratory conduit (not shown in the Figures) or directly to the atmosphere. In use, the user may exhale a portion or his or her breath via the mask assembly, that is, back into the inspiratory side of the mask assembly. In prior art masks, exhaust holes are provided in the connector 23, to assist with flushing exhaled air from the mask assembly. In the preferred form of patient interfaces, the mask body 30 has exhaust flow holes 25. The exhaust holes 25 in the mask body 30 improve the flushing of exhaled air from the assembly. The exhaust holes 25 consist of 5 to 50 holes, each hole with a diameter of 0.3mm to 1.5mm.

In a preferred form the exhaust holes 25 are provided through the front of the mask body substantially perpendicular to the front of the mask body 30. Alternatively, the exhaust holes may pass through the front of the mask body at an angle, for example, angled approximately 45 degrees upwards, the outlet of the exhaust holes at the outside surface of the mask body being higher than the inlet of the exhaust holes at the inside of the mask body.

The holes may be provided directly through the mask body 30. Alternatively, the holes may be formed in a separate rubber or plastic insert, the insert being fitted to a corresponding aperture (not shown) in the mask body 30.

The exhaust holes 25 may be provided through the front of the mask body in an upper portion of the mask body. Alternatively, the holes 25 may be provided through a side or sides of the mask body 30, in an upper portion of the mask body. Alternatively, the holes may be provided in a lower portion of the mask body, for example, adjacent a bottom edge of the mask body.

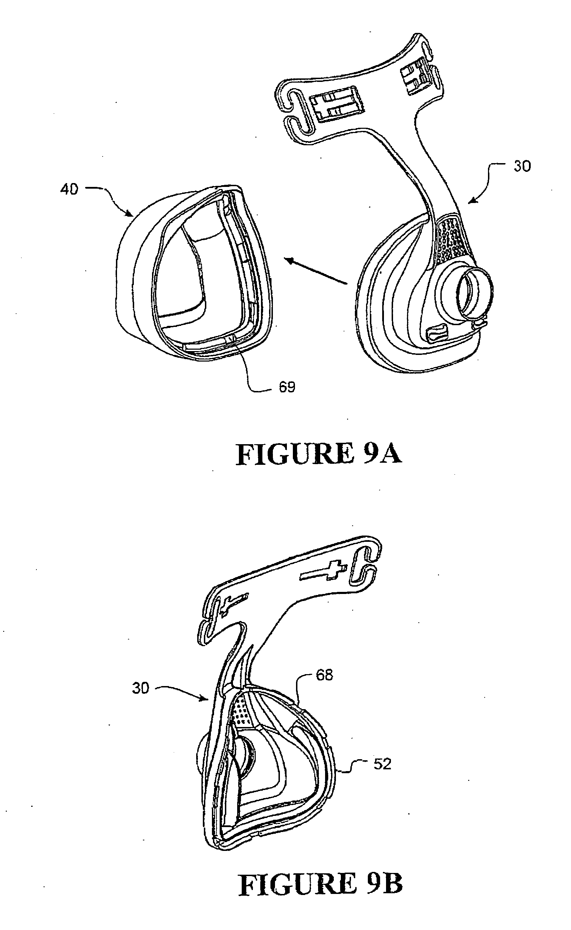

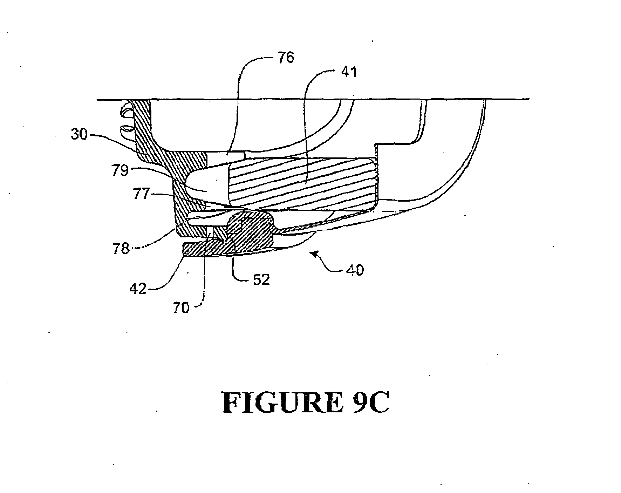

As described above, the mask body 30 preferably includes exhaust holes 25 to allow exhaled breath to be vented from the mask assembly. Alternatively, or in addition to the vent holes, the mask assembly may provide additional vent paths between the seal assembly 40 and the mask body 30, as shown in Figures 9a to 9c. The bearing surface 52 of the mask body in contact with the rim 53 or seal assembly bearing surface 51 may not extend continuously around the perimeter of the mask body. Discrete portions 68 of the rear edge of the mask body may be removed, providing a flow path from the mask assembly to the atmosphere. Alternatively, discrete portions 69 may be removed from the rim 53, or the seal assembly bearing surface 51, or both.

For clarity, discrete portions 68 and 69 have been shown enlarged in Figures 9a and 9b. In practice, the flow path via either discrete portions 68 or discrete portions 69 should be similar to the flow path via holes 25, discrete portions 68 or discrete portions 69, or both, being provided in place of holes 25.

Vent paths provided by discrete portions 68 or 69 or both are visually hidden from the exterior of the mask. This arrangement provides a desirable look to the mask assembly.

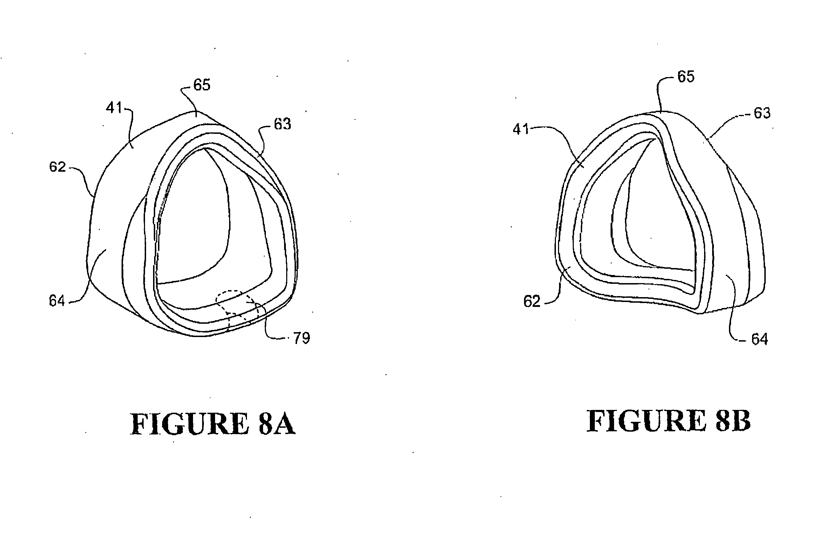

Alternatively, holes 70 through the mask body 30 adjacent to the mask bearing surface 52 may be provided to vent exhaled air. Space 78 is a part annular channel space passing around the mask, the channel being formed between the mask body 30, the mask cushion 41 and the seal assembly 40. Exhaled air passes into the channel space 78 via the raised nasal bridge region of the mask. Such holes 70 provide flow paths from the mask to the atmosphere via channel space 78. Holes 70 are provided through a side of the mask body which is partially concealed by a portion of clip 42. The holes 70 are therefore partially visually hidden from the exterior of the mask assembly in use.
In order to improve venting of exhaled air from the mask, a flow path 79 may be provided directly from the interior of the mask to the vent holes 70. In this embodiment, exhaled air does not need to pass via the raised nasal bridge region and channel space 78, but may pass directly from the interior of the mask, via flow path 79 and through holes 70. Flow path 79 is provided by a cut-out region in the mask cushion 41, indicated by the dashed line in Figure 8a. Similar cut out portions are provided in the spaced apart walls 76 and 77 which form cavity 66. The venting of exhaled air is assisted by the downwards flow path 79, with holes 70 preferably being located in a bottom side portion of the mask body 30. When a user uses the nasal interface of the present invention, air exhaled from the user's nostrils is exhaled substantially in line with the flow path 79 and holes 70, improving the vent flow path.

### Circumferential thin portion

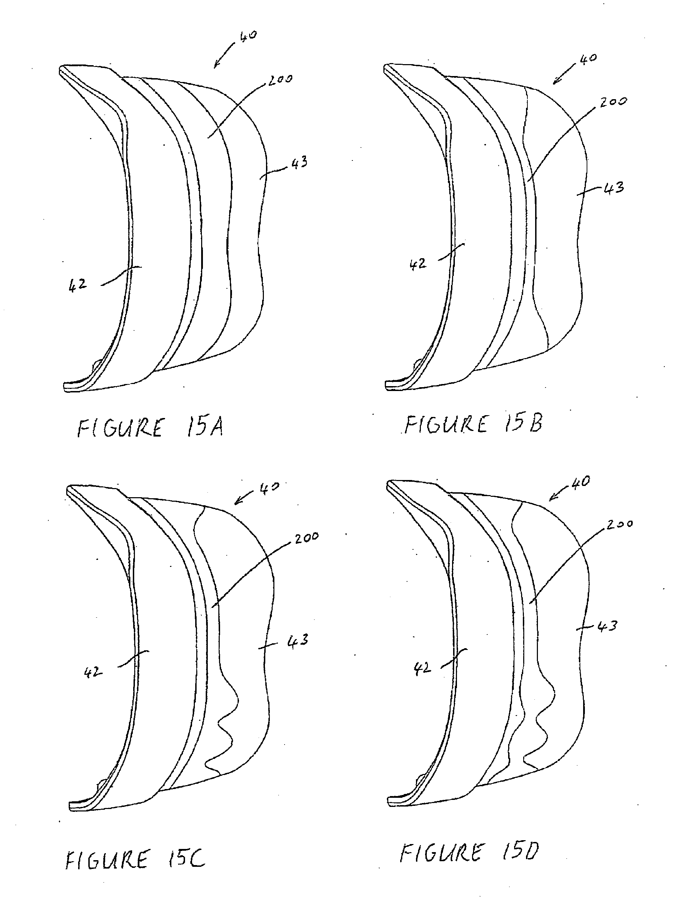

The seal 43 may comprise a thin portion approximately circumferentially aligned with the circumferential perimeter of the seal. The circumferential thin portion 200, as shown in Figures 15A to 15D, may extend continuously around the full circumference or perimeter of the seal. Alternatively, the circumferential thin portion 200 may extend around only a portion of the circumference of the seal 43.

The circumferential thin portion 200 provides flexibility in the wall of the seal. This flexibility can assist with allowing the seal area in contact with the users face to move laterally with respect to the mask body and head gear. Allowing lateral movement can assist with maintaining an effect seal against the user's face. The circumferential thin portion can provide a decoupling between the mask body and the area of the seal in contact with the user's face.

Typical CPAP pressures applied to the interior of the mask assist with providing support to the seal so that the seal 43 does not collapse in the region of the circumferential thin portion when pressure is applied between the seal and the users face by the head gear. The seal 43 may be used with or without an inner cushion 41.

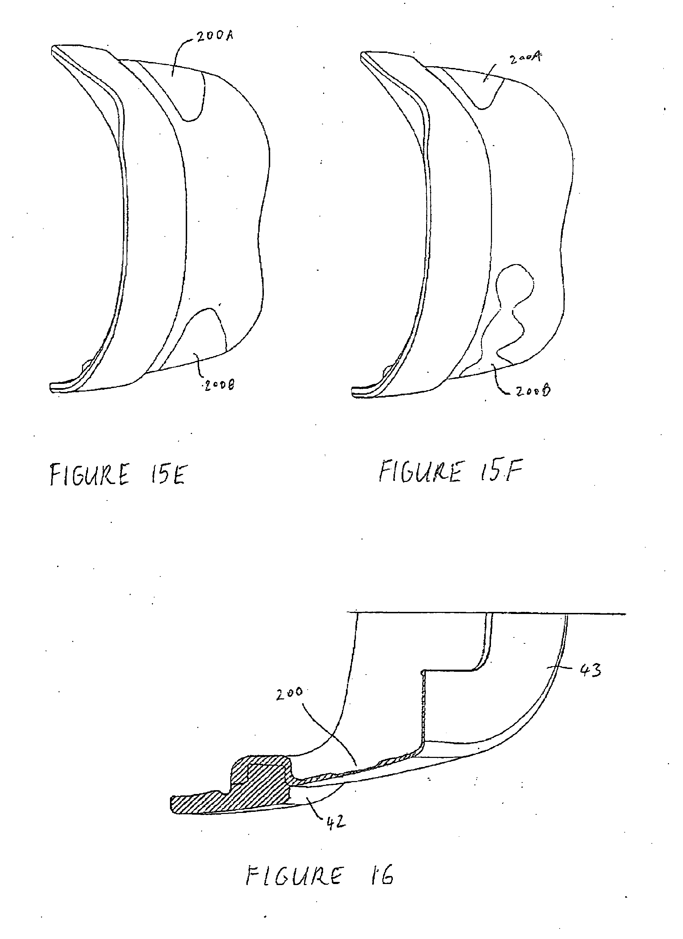

As shown in the partial cross section of Figure 16, the thin section 200 may be formed at an interior surface of the seal wall. Alternatively, the thin portion may be formed at an exterior surface of the seal wall, or may be formed centrally with respect to the cross section of the seal wall.

As shown in Figure 15A, the circumferential thin portion 200 may have approximately constant width around the circumference of the seal. Alternatively, as shown in Figure 15B, the circumferential thin portion may comprise wide portions and narrow portions. For example, the circumferential thin portion may be wider in the nasal bridge region, or the chin region or both compared to other circumferential positions around the seal.

The width of the circumferential thin portion may vary at a repeating pitch around the circumference or a portion of the circumference of the seal. For example, the width may vary at a pitch between wide and narrow sections of between approximately 10 mm to 20mm. As shown in Figures 15C and 15D, the width varies at a repeating pitch in the chin and lower check regions of the seal.

Alternatively, as shown in Figures 15E and 15F, the circumferential thin portion may comprise one or more thin portions 200A, 200B extending in a circumferential direction around the seal; thin portions 200A, 200B may be in the nasal bridge region or chin region or both.

A varying width circumferential thin portion may provide different facial sealing forces at different positions around the seal perimeter. For example, it is desirable to reduce the sealing force in the nasal bridge region. By widening the circumferential thin portion in the nasal bridge region, the seal 43 will provide less resistance in this area against the face of a user.

The circumferential thin portion may have a thickness of approximately 0.2 mm to 0.3 mm, the other sections of the seal wall having a thickness of approximately 1 mm.

The foregoing description of the invention includes preferred forms thereof. Modifications may be made thereto without departing from the scope of the invention as claimed.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

## Claims

1. A mask assembly (2) for use as part of an apparatus for supplying a flow of respiratory gases to a user comprising:
a mask body (30), said mask body including an inlet (22) through which said flow of respiratory gases are provided to the interior of said mask body, the inlet adapted to in use be connected to a gases conduit,
a mask seal assembly (40) comprising a flexible seal (43) and a clip (42), the clip being rigid relative to the flexible seal,
said seal having a first side and a second side, the first side of said seal being shaped to approximately match the contours of a user's face and in use substantially seal against a user's face, said second side for attachment to said clip,
said clip providing an interface extending substantially the full perimeter or periphery of the mask seal assembly for releasably attaching the mask seal assembly (40) to the mask body (30), and
one of the clip or the mask body having at least one recess (47) and the other one of the mask body or the clip having a series of bumps (48), interference between the bumps and the clip or the mask body during attachment of the clip to the mask body providing a positive snap type engagement of the clip to the mask body as the bumps locate in the at least one recess,
**characterized by**
one of the clip or the mask body having a lead-in section (49) providing deflection of the clip, or the mask body, or both, when attaching the clip to the mask body, the lead-in section extending at least 50% of the full perimeter of the clip or the mask body, and
said clip being shaped to generally follow the contours of a user's face, and a rear periphery (52) of said mask body also being correspondingly shaped to generally follow the contours of a user's face,
wherein the corresponding shape of the clip and the mask body, combined with the lead in section, provides for automatic correction of angular misalignment between the seal assembly and the mask body when fitting the seal assembly to the mask body.

2. The mask assembly as claimed in claim 1 wherein the seal has a wall portion (57) extending around the periphery of the seal assembly between the first and second sides, the wall being substantially constant in depth.

3. The mask assembly as claimed in claim 1 or 2 wherein the recesses (47) are located behind the lead-in section (49).

4. The mask assembly as claimed in any one of claims 1 to 3 wherein the lead-in section (49) has a length to height ratio of 3 to 7.

5. The mask assembly as claimed in any one of claims 1 to 4 wherein a cross section of the mask seal assembly at the clip (42) has a width to height ratio of 1.5 to 2.

6. The mask assembly as claimed in claim 5 wherein the clip (42) is adapted to disengage the seal assembly (40) from the mask body (30) when squeezed at opposite perimeter portions at positions absent of a said bump (48).

7. The mask assembly as claimed in any one of claims 1 to 6 wherein the seal assembly (40) attaches to the mask body (30) in a sealed engagement, a first bearing surface (51) of the seal bearing against a corresponding second bearing surface (52) of the mask body in a butting engagement.

8. The mask assembly as claimed in claim 7 wherein the first bearing surface comprising a raised rim (45), in use the raised rim being compressed against the second bearing surface.

9. The mask assembly as claimed in any one of claims 1 to 8 wherein the clip has at least one wing portion (44), the wing portion providing a gripping flange to assist with removing the seal assembly (40) from the mask body (30).

10. The mask assembly as claimed in any one of claims 1 to 9 wherein the lead-in section (49) extends substantially the full perimeter of the seal assembly clip or the mask body.

11. The mask assembly as claimed in any one of claims 1 to 10 wherein the mask assembly further comprises headgear (21) for securing the mask assembly to the head of a user.

12. The mask assembly as claimed in any one of claims 1 to 11 wherein the mask assembly further comprises an inner cushion (41) around the outer periphery of the mask body between the mask body and the first side of the seal.

## Patentansprüche

1. Maskenanordnung (2) zur Verwendung als Teil einer Vorrichtung zur Zuführung eines Stroms von Atemgasen zu einem Benutzer, umfassend:
einen Maskenkörper (30), wobei der Maskenkörper einen Einlass (22) enthält, durch den der Strom von Atemgasen dem Inneren des Maskenkörpers zugeführt wird, wobei der Einlass dazu ausgeführt ist, im Gebrauch mit einer Gasleitung verbunden zu werden,
eine Maskendichtungsanordnung (40), die eine flexible Dichtung (43) und eine Klammer (42) umfasst, wobei die Klammer bezüglich der flexiblen Dichtung starr ist,
wobei die Dichtung eine erste Seite und eine zweite Seite aufweist, wobei die erste Seite der Dichtung so geformt ist, dass sie ungefähr mit den Konturen des Gesichts eines Benutzers zusammenpasst und im Gebrauch gegen das Gesicht eines Benutzers im Wesentlichen abdichtet, wobei die zweite Seite der Befestigung der Klammer dient,
wobei die Klammer eine sich im Wesentlichen um den ganzen Perimeter oder Umfang der Maskendichtungsanordnung erstreckende Grenzfläche zur lösbaren Befestigung der Maskendichtungsanordnung (40) an dem Maskenkörper (30) bereitstellt, und
wobei die Klammer oder der Maskenkörper mindestens eine Aussparung (47) aufweist und das jeweils andere Element, der Maskenkörper oder die Klammer, eine Reihe von Höckern (48) aufweist, wobei eine gegenseitige Beeinflussung zwischen den Höckern und der Klammer oder dem Maskenkörper während der Befestigung der Klammer an dem Maskenkörper einen formschlüssigen Rasteingriff der Klammer mit dem Maskenkörper bereitstellt, wenn sich die Höcker in der mindestens einen Aussparung positionieren,
**dadurch gekennzeichnet, dass**
die Klammer oder der Maskenkörper einen Einführungsabschnitt (49) aufweist, der eine Durchbiegung der Klammer und/oder des Maskenkörpers bei Befestigung der Klammer an dem Maskenkörper bereitstellt, wobei sich der Einführungsabschnitt über mindestens 50% des gesamten Perimeters der Klammer oder des Maskenkörpers erstreckt, und
die Klammer so geformt ist, dass sie den Konturen des Gesichts eines Benutzers allgemein folgt, und ein hinterer Umfang (52) des Maskenkörpers auch entsprechend so geformt ist, dass er den Konturen des Gesichts eines Benutzers allgemein folgt,
wobei die entsprechende Form der Klammer und des Maskenkörpers, zusammen mit dem Einführungsabschnitt, eine automatische Korrektur einer Winkelfehlausrichtung zwischen der Dichtungsanordnung und dem Maskenkörper bei Anordnung der Dichtungsanordnung an dem Maskenkörper gewährleistet.

2. Maskenanordnung nach Anspruch 1, wobei die Dichtung einen Wandteil (57) aufweist, der sich um den Umfang der Dichtungsanordnung zwischen der ersten und der zweiten Seite erstreckt, wobei die Wand eine im Wesentlichen konstante Tiefe aufweist.

3. Maskenanordnung nach Anspruch 1 oder 2, wobei die Aussparungen (47) hinter dem Einführungsabschnitt (49) positioniert sind.

4. Maskenanordnung nach einem der Ansprüche 1 bis 3, wobei der Einführungsabschnitt (49) ein Verhältnis von Länge zu Höhe von 3 zu 7 aufweist.

5. Maskenanordnung nach einem der Ansprüche 1 bis 4, wobei ein Querschnitt der Maskendichtungsanordnung an der Klammer (42) ein Verhältnis von Breite zu Höhe von 1,5 zu 2 aufweist.

6. Maskenanordnung nach Anspruch 5, wobei die Klammer (42) dazu ausgeführt ist, die Dichtungsanordnung (40) aus dem Maskenkörper (30) auszurücken, wenn sie an einander gegenüberliegenden Perimeterteilen an Stellen ohne einen solchen Höcker (48) zusammengedrückt wird.

7. Maskenanordnung nach einem der Ansprüche 1 bis 6, wobei die Dichtungsanordnung (40) in einem abgedichteten Eingriff an dem Maskenkörper (30) befestigt ist, wobei eine erste Lagerfläche (51) der Dichtung gegen eine entsprechende zweite Lagerfläche (52) des Maskenkörpers in einem aneinander anstoßenden Eingriff drückt.

8. Maskenanordnung nach Anspruch 7, wobei die erste Lagerfläche einen erhabenen Rand (45) umfasst, wobei der erhabene Rand im Gebrauch gegen die zweite Lagerfläche komprimiert wird.

9. Maskenanordnung nach einem der Ansprüche 1 bis 8, wobei die Klammer mindestens einen Flügelteil (44) aufweist, wobei der Flügelteil einen Greifflansch bereitstellt, um das Entfernen der Dichtungsanordnung (40) von dem Maskenkörper (30) zu unterstützen.

10. Maskenanordnung nach einem der Ansprüche 1 bis 9, wobei sich der Einführungsabschnitt (49) im Wesentlichen über den ganzen Perimeter der Dichtungsanordnungsklammer oder des Maskenkörpers erstreckt.

11. Maskenanordnung nach einem der Ansprüche 1 bis 10, wobei die Maskenanordnung weiterhin ein Kopfgeschirr (21) zur Befestigung der Maskenanordnung am Kopf eines Benutzers umfasst.

12. Maskenanordnung nach einem der Ansprüche 1 bis 11, wobei die Maskenanordnung weiterhin ein Innenpolster (41) um den Außenumfang des Maskenkörpers zwischen dem Maskenkörper und der ersten Seite der Dichtung umfasst.

## Revendications

1. Ensemble (2) masque destiné à être utilisé comme partie d'un appareil pour alimenter un flux de gaz respiratoires à un utilisateur comportant :
un corps (30) de masque, ledit corps de masque comprenant un orifice (22) d'entrée dans lequel ledit flux de gaz respiratoires est fourni à l'intérieur dudit corps de masque, l'orifice d'entrée étant conçu en fonctionnement pour être raccordé à un conduit de gaz,
un ensemble (40) joint d'étanchéité de masque comportant un joint (43) d'étanchéité flexible et une pince (42), la pince étant rigide par rapport au joint d'étanchéité flexible,
ledit joint d'étanchéité présentant une première face et une seconde face, la première face dudit joint d'étanchéité ayant une forme conçue pour correspondre à peu près aux contours du visage d'un utilisateur et en fonctionnement pour venir sensiblement se plaquer hermétiquement contre le visage d'un utilisateur, ladite deuxième face étant conçue pour être attachée à ladite pince,
ladite pince fournissant une interface s'étendant sensiblement sur tout le pourtour ou la périphérie de l'ensemble élément d'étanchéité de masque pour attacher de manière libérable l'ensemble (40) joint d'étanchéité de masque au corps (30) de masque, et
la pince ou le corps de masque étant pourvu d'au moins un renfoncement (47) et l'autre soit corps de masque soit pince étant pourvu d'une série de bosses (48), l'entrave entre les bosses et la pince ou le corps de masque au cours de l'attache de la pince au corps du masque fournissant un contact positif par encliquetage de la pince avec le corps de masque au fur et à mesure que les bosses sont positionnées dans ledit au moins un renfoncement,
**caractérisé en ce que**
la pince ou le corps de masque est pourvu d'une première section (49) fournissant une déformation de la pince, ou du corps de masque, ou des deux, lorsque la pince est attachée au corps de masque, la première section s'étendant sur au moins 50 % de la totalité du pourtour de la pince ou du corps de masque, et
ladite pince ayant une forme qui suit globalement les contours du visage d'un utilisateur, et une périphérie (52) arrière dudit corps de masque ayant aussi, de manière correspondante, une forme qui suit globalement les contours du visage d'un utilisateur,
dans lequel la forme correspondante de la pince et du corps de masque, combinée à la première section, fournit une correction automatique du mauvais alignement angulaire entre l'ensemble joint d'étanchéité et le corps de masque lorsque l'ensemble joint d'étanchéité est monté sur le corps de masque.

2. Ensemble masque selon la revendication 1 dans lequel le joint d'étanchéité présente une partie (57) de paroi qui s'étend autour de la périphérie de l'ensemble élément joint d'étanchéité entre les première et deuxième faces, la paroi ayant une profondeur sensiblement constante.

3. Ensemble masque selon la revendication 1 ou 2 dans lequel les renfoncements (47) sont positionnés derrière la première section (49).

4. Ensemble masque selon l'une quelconque des revendications 1 à 3 dans lequel la première section (49) a un rapport longueur-hauteur de 3 à 7.

5. Ensemble masque selon l'une quelconque des revendications 1 à 4 dans lequel une section en coupe de l'ensemble joint d'étanchéité de masque au niveau de la pince (42) a un rapport largeur-hauteur de 1,5 à 2.

6. Ensemble masque selon la revendication 5 dans lequel la pince (42) est conçue pour désolidariser l'ensemble (40) joint d'étanchéité du corps (30) de masque lorsque l'on appuie sur des parties opposées du pourtour à des endroits où ladite bosse (48) est absente.

7. Ensemble masque selon l'une quelconque des revendications 1 à 6 dans lequel l'ensemble (40) joint d'étanchéité s'attache au corps (30) de masque en un contact hermétique, une première surface (51) d'appui du joint d'étanchéité s'appuyant contre une seconde surface (52) d'appui correspondante du corps de masque en un contact par aboutement.

8. Ensemble masque selon la revendication 7 dans lequel la première surface d'appui comporte un rebord (45) surélevé, le rebord surélevé étant, en fonctionnement, comprimé contre la seconde surface d'appui.

9. Ensemble masque selon l'une quelconque des revendications 1 à 8 dans lequel la pince présente au moins une partie (44) ailette, la partie ailette fournissant une bride de saisie pour aider à enlever l'ensemble (40) joint d'étanchéité du corps (30) de masque.

10. Ensemble masque selon l'une quelconque des revendications 1 à 9 dans lequel la première section (49) s'étend sensiblement sur tout le pourtour de la pince dans l'ensemble joint d'étanchéité ou le corps de masque.

11. Ensemble masque selon l'une quelconque des revendications 1 à 10 dans lequel l'ensemble masque comporte en outre un casque (21) pour fixer l'ensemble masque sur la tête d'un utilisateur.

12. Ensemble masque selon l'une quelconque des revendications 1 à 11 dans lequel l'ensemble masque comporte en outre un coussin (41) intérieur autour de la périphérie extérieure du corps de masque entre le corps de masque et la première face du joint d'étanchéité.
